# EUROPEAN PATENT SPECIFICATION

(11) **EP 0 379 572 B1**
(45) Date of publication and mention of the grant of the patent: **15.06.1994**
(21) Application number: 89909058.3
(22) Date of filing: 07.07.1989
(51) Int. Cl.: A61L 2/00, A61M 1/36

(54) **METHOD OF TREATING BLOOD**
BLUTBEHANDLUNG
PROCEDE DE TRAITEMENT DU SANG

(30) Priority: 14.07.1988 US 219331
(43) Date of publication of application: 01.08.1990
(73) Proprietor: LIBERMAN, Barnet, L, New York, NY 10014 (US)
(72) Inventor: LIBERMAN, Barnet, L, New York, NY 10014 (US)
(74) Representative: Gustorf, Gerhard, Dipl.-Ing.
(86) International application number: US8902991
(87) International publication number: WO9000409

(56) References cited:
- FR-A- 1 356 498
- US-A- 3 344 617
- US-A- 3 856 475
- US-A- 4 339 928
- US-A- 4 601 909
- US-A- 4 654 217
- US-A- 4 657 768
- US-A- 4 787 883
- US-A- 4 840 034

## Description

This invention relates generally to methods of treating blood and, in particular, to a method of treating blood which inactivates cold sensitive viruses or other organisms contained in the blood.

Methods of treating body fluids by extracting the fluid from a patient requiring treatment are known and available. For example, in a kidney dialysis process metabolic waste products are removed from a human body and cleansed so as to perform the function ordinarily performed by the kidneys. Other processes including treatment of blood using ultraviolet light to alleviate certain diseases are also becoming known.

Furthermore, it is known that certain organisms which survive in blood are inactivated at low temperatures. In particular, the HIV virus which causes acquired immune deficiency syndrome (AIDS) does not survive at temperatures less than about 24°C (75°F). Accordingly, if blood containing the HIV virus were removed from a human body where it is maintained at a body temperature of about 37°C (98.6°F.) and chilled to a temperature at which the virus does not survive, it is believed that active HIV virus would no longer be present in the blood.

The difficulty with this approach is that most processes for chilling blood are expensive, time consuming or destroy the cellular structure of the blood. For example, storage of blood in a frozen condition until it is needed by a patient requiring a blood donation is known. However, this practice is generally not followed since blood banks rarely have an over-supply of blood requiring techniques for extended storage and cryogenic techniques for freezing blood which primarily use liquid nitrogen are expensive. It has been thought that if blood could be chilled using an economical process, cold-sensitive viruses could be inactivated, the cellular structure of the blood could be maintained intact, and the process could be readily used as a standard procedure.

U.S. Patent No. 4,601,909 issued to Nagoshi on July 22, 1986, discloses a Method of Freezing Fishery Products. The method includes the steps of preparing a brine containing rapeseed oil, propylene glycol, calcium chloride and water, cooling the brine and immersing the seafood in the cooled brine until it is frozen. The rapeseed oil is said to increase the freezing rate of the seafood and reduce the time required to carry out the freezing process. Breakdown of muscle tissue in the seafood due to ice crystal formation and deterioration in quality is prevented.

A similar process for Quick Freezing of Meat is disclosed and claimed in U.S. patent No. 4,654,217 issued to Nagoshi on March 31, 1987. The process disclosed in this later patent is similar to that disclosed in the earlier patent except that it is applicable to beef, poultry, pork and the like.

U.S. patent No. 4,657,768 issued to Nagoshi on April 14, 1987, discloses a Freezing Method for Perishable Foods which includes placing a perishable food in a heat conducting container and causing the other surface of the heat conducting container to contact cooled brine or a liquified gas. Accordingly, the perishable food is frozen quickly without immersion.

U.S. patent No. 4,689,963 issued to Sakai on September 1, 1987, relates to a Method of Freezing Foods. The method of Sakai is similar to the method of Nagoshi except that a layer of brine is placed in the heat conducting container along with the perishable food. Freezing proceeds only from the portion which is in contact with the brine and the layer of brine prevents or reduces the tendency of the food to stick to the container.

There is no teaching or suggestion in any of these patents that these processes can be used to chill blood in order to inactivate cold-sensitive organisms.

Accordingly, it is desirable to provide a process for chilling blood to inactivate cold sensitive viruses or other organisms contained therein by a method which does not deteriorate the cellular structure.

It is, therefore, an object of the invention is to provide a rapid method of treating blood to inactivate cold sensitive organisms.

Another object of the invention is to provide a method of inactivating cold sensitive viruses and other organisms in blood by chilling the blood using a brine including a suitable oil.

A further object of the invention is to provide a method of treating blood to inactivate cold sensitive viruses or other organisms that can be performed as a continuous process or as a batch process.

Still another object of the invention is to provide an economical method of treating blood to inactivate cold-sensitive organisms.

Still other objects and advantages of the invention will in part be obvious and will in part be apparent from the specification.

### SUMMARY OF THE INVENTION

Generally speaking, in accordance with the invention a method for treating blood of a mammalian subject in order to inactivate cold sensitive viruses and other organisms in the blood is provided. The method includes the steps of preparing a brine including a suitable oil; cooling the brine to a temperature between about -30°C (-22°F) and -42°C (-43.6°F.); extracting blood thought to contain at least one strain of cold sensitive virus or other organism from the body of a mammalian subject; subjecting the extracted blood to the cooled brine for a period of time sufficient to chill the blood and inactivate the virus or other organism; warming the chilled blood to a temperature at which it can be reintroduced into the mammalian subject; and reintroducing the warmed blood into the body of the same mammalian subject from which it was extracted. The brine generally includes a glycol, a salt and water and the method can be performed either as a continuous or a batch process.

The invention accordingly comprises the several steps and the relation of one or more of such steps with respect to each of the others thereof which will be exemplified in the method hereinafter disclosed and the scope of the invention will be indicated in the claims.

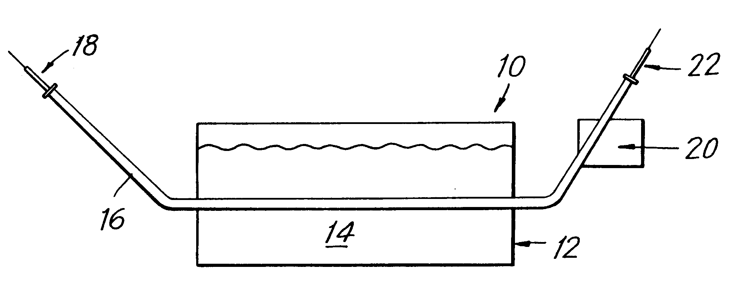

### DESCRIPTION OF THE DRAWING

For a fuller understanding of the invention, reference is made to the following description taken in connection with the accompanying drawing in which the Figure is a diagrammatic view of an apparatus constructed and arranged for use in accordance with the method of the invention.

### DETAILED DESCRIPTION OF THE PREFERRED EMBODIMENTS

It is well known that certain cold sensitive viruses and other organisms can be present in mammalian blood. One cold sensitive virus of exceptional concern is the HIV virus, the virus which is known to cause acquired immune deficiency syndrome, commonly known as AIDS. Although AIDS is inevitably a fatal disease, the HIV virus itself is fragile and becomes inactive at temperatures less than about 24°C (75°F). Accordingly, if blood containing the HIV virus were removed from the body of a mammalian subject, especially a human, and chilled to a temperature of less than about 24°C (75°F.) by the process described below, the virus would become inactive in the blood. The blood could then be reintroduced into the body of the mammalian subject.

The first step of the process of treating blood in accordance with the invention is preparation of a suitable brine solution which includes a cruciferous oil. In a preferred embodiment, oil from a plant of the genus Brassica is used. These oils include, but are not limited to, oil of Brassica campestris, otherwise known as rapeseed oil, and oil of Brassica hirta, also known as mustard oil.

Rapeseed oil has a solidification point of 14°F. (-10°C.), a specific gravity at 59°F. (15°C.) of 0.915, a refractive index at 122°F. (50°C.) of 1.4706, an iodine value of 98.6 and a saponification value of 174.7. The oil includes about 1% palmitic acid, the only saturated component of the oil, about 32% oleic acid, about 15% linoleic acid, about 1% linolenic acid and about 50% erucic acid. Palmitic acid, otherwise known as hexadecanoic acid, has 16 carbon atoms and a molecular weight of 256.4.

Oleic acid, also known as (Z)-9-octadecenoic acid, has 18 carbon atoms and a molecular weight of 282.5. The position of unsaturation is between the ninth and tenth carbon atoms in the chain. The molecule has a cis configuration.

Linoleic acid has two positions of unsaturation and is also known as cis,cis-9,12-octadecadienoic acid. The acid has 18 carbon atoms and a molecular weight of 280.5.

Linolenic acid has three positions of unsaturation and is also known as (Z,Z,Z)-9,12,15-octadecatrienoic acid. Linolenic acid has 18 carbon atoms and a molecular weight of 278.4.

Erucic acid, a major component of the oils of the genus Brassica, is also known as (Z)-13-docosanoic acid. Erucic acid has 22 carbon atoms with one position of unsaturation and a molecular weight of 338.6.

Mustard oil is similar and has a specific gravity at 59°F. (15°C) of 0.9145, a refractive index at 122°F. (50°C) of 1.475, an iodine value of 102 and a saponification value of 174. Mustard oil includes 1.3% by weight myristic acid, the only saturated acid, 27.2% by weight oleic acid, 16.6% by weight linoleic acid, 1.8% by weight linolenic acid, 1.1% by weight behenic acid, 1.0% by weight lignoceric acid and 51.0% by weight erucic acid. Myristic acid, also known as tetradecanoic acid, has 14 carbon atoms and a molecular weight of 228.4.

Behenic acid is also known as docosanoic acid. It has 22 carbon atoms and a molecular weight of 340.6. Lignoceric acid, also known as tetracosanoic acid, has 24 carbon atoms and a molecular weight of 368.6. The other components of mustard oil are described above.

The oil is used in an amount less than about 1% weight, more preferably less than about 0.8% by weight and most preferably between 0.1 and 0.5% by weight of the brine. The oil is used to increase the freezing rate of blood immersed in the brine when the brine has been cooled to between about -30°C (-22°F) and - -42°C (43.6°F). Consequently, deterioration of the cellular structure of the blood is minimized.

It is to be understood that oils other than rapeseed oil and mustard oil can be used in accordance with the invention. For example, synthetic oils having the characteristics described would be useful. In addition, the manner in which the oils function is described in detail below and it will be readily apparent that other oils will function acceptably in accordance with the invention and can be readily determined.

In addition to the cruciferous oil, the brine also generally includes a glycol, an inorganic salt and water. Suitable glycols include, but are not limited to, ethylene glycol, propylene glycol, benzylene glycol, butylene glycol, diethylene glycol, diphenyl glycol, ethylidene glycol, and the like. Any glycol can be used alone or in combination with other glycols. Propylene glycol is used in a preferred embodiment. The glycol component is present in an amount between about 30 and 50% by weight of the brine, more preferably between about 35 and 45% by weight and most preferably in an amount of about 40% by weight.

Salts which are useful in accordance with the invention include, but are not limited to, calcium chloride, calcium bromide, calcium iodide, potassium chloride, potassium bromide, potassium iodide and the like. In a preferred embodiment, calcium chloride is used. The salt is present in an amount between about 5 and 15% by weight of the brine, more preferably in an amount between about 7 and 13% by weight and most preferably in an amount of about 10% by weight.

Water is present in an amount between about 40 and 60% by weight, more preferably in an amount between about 45 and 55% by weight and most preferably in an amount of about 50% by weight.

The brine including the cruciferous oil is cooled to a temperature between about -30°C (-22°F) and - -42°C (43.6°F). It is presently believed that this causes fine ice crystals to form and be uniformly distributed in the brine. Assuming that the assumption regarding the ice crystals is correct, it is further believed that the crystals permit efficient cold transfer and an increase in the expected chilling rate of blood brought into a heat transfer relationship with the brine. Consequently, the time required for cooling the blood is reduced. In a preferred embodiment, means are provided for removing heat from the brine so that the temperature of the brine remains substantially constant when blood at a body temperature of about 37°C (98.6°F.) is introduced.

Blood containing a cold-sensitive organism such as the HIV virus is extracted through a needle from the body of a mammalian, generally human, subject. The extracted blood passes, for example, from the needle and through tubing which extends through a suitable brine as shown in the Figure.

Referring to the Figure, a diagram of a chilling apparatus 10 constructed and arranged for use in accordance with the method of the invention is depicted. Chilling apparatus 10 includes a basin 12 adapted to contain a brine 14 therein. A length of tubing 16, which may be plastic or the like, extends from a needle 18 through which blood is extracted through brine 14 and then to a warming means 20 for warming the blood to a temperature at which it can be reintroduced into a mammalian body, nominally 37°C (98.6°F.) for a human. Tubing 16 then connects to a second needle 22 through which it can be re-introduced into a mammalian subject.

As can be seen, this apparatus is useful for a continuous process where the blood is chilled, but not frozen. Freezing of the blood would inhibit flow through the brine. Accordingly, it is necessary to adjust the length of tubing through the brine so that the blood remains in a liquid state.

In an alternate embodiment, blood is extracted from a body and collected in commonly available plastic bags of a type which are well known in the art. Each plastic bag may have a capacity of about 1/2 pint in accordance with generally accepted medical practice.

As a plastic bag is filled with blood, it is removed from the tubing and replaced with another bag. The bag which has been removed is then immersed in the cooled brine for a period of less than about 2 minutes, preferably between about 30 seconds and 1 1/2 minutes in order to chill the blood and inactivate cold-sensitive viruses contained therein. Since some blood is in the chilling process while other blood is being withdrawn from the body, it is important to monitor the process so that only a medically appropriate amount of blood is absent from the body at any given time.

In an alternate method, which is useful in accordance with the invention, the blood can be chilled by placement in a heat-conducting pan or tray. The opposite side of the pan or tray is then placed in contact with the cooled brine described. In a further alternate embodiment, brine is placed in the heat-conducting pan or tray along with a container of blood and then the opposite side of the tray is placed into contact with the cooled brine in order to chill the blood samples.

The chilled blood is warmed to body temperature for reintroduction into the body. Blood chilled by this process can be warmed fairly rapidly, thereby further preventing cellular tissue breakdown.

It is to be understood that although a continuous process for extracting and chilling blood has been described, the process can be performed as a batch process. For example, a specific quantity of blood could be extracted, such as a pint, and chilled. The pint of blood is then reintroduced into the body of the mammalian subject.

In fact, it is not necessary to reintroduce the blood into the body of the same mammalian subject. For example, the process would to be useful for inactivating live cold sensitive viruses and may, in fact, be useful for maintaining the integrity of blood supplies stored in blood banks.

It is also to be understood that the process can be performed using either manual or automated equipment and the scope of the invention is not intended to be limited by the type of equipment on which the process is performed.

Additionally, although the process has been described as applicable for treating persons infected with the HIV or AIDS-causing virus, it is to be understood that the process is useful for inactivating most viral organisms, since such organisms generally do not survive at sub-freezing temperatures.

It will thus be seen that the objects set forth above, among those made apparent from the preceding description, are efficiently attained and, since certain changes may be made in carrying out the above method without departing from the spirit and scope of the invention, it is intended that all matter contained in the above description shall be interpreted as illustrative and not in a limiting sense.

It is also to be understood that the following claims are intended to cover all of the generic and specific features of the invention herein described and all statements of the scope of the invention which, as a matter of language, might be said to fall therebetween.

Particularly, it is to be understood that in said claims, ingredients or compounds recited in the singular are intended to include compatible mixtures of such ingredients wherever the sense permits.

## Claims

1. Method of treating blood extracted from the body of a mammalian subject and containing at least one cold sensitive organism, comprising:
- preparing a brine including a cruciferous oil in an amount between 0,1 % and 1 % weight,
- cooling the brine to a temperature between -30°C (-22°F) and -42°C (-43,6°F),
- subjecting the extracted blood to a heat transfer relationship with the cooled brine for a period of less than 2 minutes.

2. Method according to claim 1, wherein the oil is extracted from a plant of the genus Brassica.

3. Method according to claim 1, wherein the oil is selected from the group consisting of rapeseed oil, mustard oil and mixtures thereof.

4. Method according to anyone of the preceding claims, wherein the oil contains erucic acid as the single largest component.

5. Method according to anyone of the preceding claims, wherein the oil contains less than 2% saturated components.

6. Method according to anyone of the preceding claims, wherein the oil is used in an amount between 0,1 and 0,5% by weight of the brine.

7. Method according to anyone of the preceding claims, wherein the brine further includes a glycol, an inorganic salt and water.

8. Method according to claim 7, wherein the glycol is propylene glycol.

9. Method according to claim 7 or 8, wherein the glycol is present in an amount between 30% and 50% by weight of the brine.

10. Method according to anyone of claims 7 to 9, wherein the salt is calcium chloride.

11. Method according to anyone of claims 7 to 10, wherein the salt is present in an amount between 5% and 15% by weight of the brine.

12. Method according to anyone of claims 7 to 11, wherein the water is present in an amount between 40% and 60% by weight of the brine.

13. Method according to anyone of the preceding claims, further including warming the chilled blood to the body temperature of a mammalian subject.

14. Method according to claim 13, wherein the chilled blood is warmed to a temperature of 37°C (98,6°F).

15. Method according to anyone of the preceding claims, wherein the cold sensitive organism is HIV virus and the mammalian subject is a human.

16. Method according to anyone of the preceding claims, wherein the process is performed as a continuous process.

17. Method according to anyone of claims 1 to 15, wherein the process is performed as a batch process.

## Patentansprüche

1. Verfahren zur Behandlung von Blut, das dem Körper eines Mammaliers entnommen ist und wenigstens einen kälteempfindlichen Organismus enthält, wobei
- eine Sole zubereitet wird, die ein Kruziferenöl in einer Menge zwischen 0,1 und 1 Gew.% enthält,
- die Sole auf eine Temperatur zwischen - 30° C und - 42° C abgekühlt wird und
- das entnommene Blut gegenüber der gekühlten Sole einem Wärmeaustausch von weniger als 2 Minuten ausgesetzt wird.

2. Verfahren nach Anspruch 1, dadurch gekennzeichnet, daß das Öl einer Pflanze der Gattung Brassica entnommen ist.

3. Verfahren nach Anspruch 1, dadurch gekennzeichnet, daß das Öl aus der Gruppe ausgewählt ist, die Rapsöl, Senföl oder deren Mischungen enthält.

4. Verfahren nach einem der vorhergehenden Ansprüche, dadurch gekennzeichnet, daß das Öl Erucasäure als die einzige große Komponente enthält.

5. Verfahren nach einem der vorhergehenden Ansprüche, dadurch gekennzeichnet, daß das Öl weniger als 2% gesättigte Bestandteile hat.

6. Verfahren nach einem der vorhergehenden Ansprüche, dadurch gekennzeichnet, daß das Öl in einer Menge zwischen 0,1 und 0,5 Gew.% der Sole verwendet wird.

7. Verfahren nach einem der vorhergehenden Ansprüche, dadurch gekennzeichnet, daß die Sole ferner Glykol, ein anorganisches Salz und Wasser enthält.

8. Verfahren nach Anspruch 7, dadurch gekennzeichnet, daß das Glykol Propylenglykol ist.

9. Verfahren nach Anspruch 7 oder 8, dadurch gekennzeichnet, daß das Glykol in einem Anteil zwischen 30 und 50 Gew.% der Sole verwendet wird.

10. Verfahren nach einem der Ansprüche 7 bis 9, dadurch gekennzeichnet, daß das Salz Kalziumchlorid ist.

11. Verfahren nach einem der Ansprüche 7 bis 10, dadurch gekennzeichnet, daß das Salz in einem Anteil zwischen 5 und 15 Gew.% der Sole verwendet wird.

12. Verfahren nach einem der Ansprüche 7 bis 11, dadurch gekennzeichnet, daß Wasser in einem Anteil zwischen 40 und 60 Gew.% der Sole verwendet wird.

13. Verfahren nach einem der vorhergehenden Ansprüche, dadurch gekennzeichnet, daß das abgekühlte Blut wieder auf die Körpertemperatur des Mammaliers erwärmt wird.

14. Verfahren nach Anspruch 13, dadurch gekennzeichnet, daß das abgekühlte Blut auf eine Temperatur von 37° C erwärmt wird.

15. Verfahren nach einem der vorhergehenden Ansprüche, dadurch gekennzeichnet, daß der kälteempfindliche Organismus ein HIV-Virus und der Mammalier ein Mensch ist.

16. Verfahren nach einem der vorhergehenden Ansprüche, dadurch gekennzeichnet, daß dieses als kontinuierlicher Prozeß durchgeführt wird.

17. Verfahren nach einem der Ansprüche 1 bis 15, dadurch gekennzeichnet, daß dieses als diskontinuierlicher Prozeß durchgeführt wird.

## Revendications

1. Procédé de traitement du sang extrait du corps d'un sujet mammifère et contenant au moins un organisme sensible au froid, comprenant les étapes consistant à:
- préparer une saumure incluant une huile de crucifère selon une proportion comprise entre 0,1% et 1% en poids,
- refroidir la saumure à une température comprise entre -30°C (-22°F) et -42°C (-43,6°F),
- soumettre le sang extrait à une relation de transfert thermique avec la saumure refroidie pendant une période de moins de 2 minutes.

2. Procédé selon la revendication 1, dans lequel l'huile est extraite d'une plante du genre Brassica.

3. Procédé selon la revendication 1, dans lequel l'huile est choisie dans le groupe constitué par l'huile de colza, l'huile de moutarde et des mélanges de celles-ci.

4. Procédé selon l'une quelconque des revendications précédentes, dans lequel l'huile contient de l'acide érucique comme composant individuel le plus important.

5. Procédé selon l'une quelconque des revendications précédentes, dans lequel l'huile contient moins de 2% de composants saturés.

6. Procédé selon l'une quelconque des revendications précédentes, dans lequel l'huile est utilisée selon une proportion comprise entre 0,1% et 0,5% en poids de la saumure.

7. Procédé selon l'une quelconque des revendications précédentes, dans lequel la saumure inclut en outre un glycol, un sel inorganique et de l'eau.

8. Procédé selon la revendication 7, dans lequel le glycol est le propylène glycol.

9. Procédé selon la revendication 7 ou 8, dans lequel le glycol est présent selon une proportion comprise entre 30% et 50% en poids de la saumure.

10. Procédé selon l'une quelconque des revendications 7 à 9, dans lequel le sel est du chlorure de calcium.

11. Procédé selon l'une quelconque des revendications 7 à 10, dans lequel le sel est présent selon une proportion comprise entre 5% et 15% en poids de la saumure.

12. Procédé selon l'une quelconque des revendications 7 à 11, dans lequel l'eau est présente selon une proportion comprise entre 40% et 60% en poids de la saumure.

13. Procédé selon l'une quelconque des revendications précédentes, comprenant en outre l'étape consistant à chauffer, à la température du corps d'un sujet mammifère, le sang refroidi.

14. Procédé selon la revendication 13, dans lequel le sang refroidi est chauffé à une température de 37°C (98,6°F).

15. Procédé selon l'une quelconque des revendications précédentes, dans lequel l'organisme sensible au froid est un virus HIV et le sujet mammifère est un humain.

16. Procédé selon l'une quelconque des revendications précédentes, dans lequel le processus est effectué sous forme de processus continu.

17. Procédé selon l'une quelconque des revendications 1 à 15, dans lequel le processus est effectué sous forme de processus par lots.
